# EUROPÄISCHE PATENTSCHRIFT

(11) **EP 0 909 182 B1**
(45) Veröffentlichungstag und Bekanntmachung des Hinweises auf die Patenterteilung: **20.02.2002**
(21) Anmeldenummer: 97931715.3
(22) Anmeldetag: 04.07.1997
(51) Int. Cl.: A61K 41/00, A61K 47/48

(54) **HERSTELLUNG POLYETHER-AUFWEISENDER CHLORINE UND BAKTERIOCHLORINE**
METHOD FOR PRODUCING CHLORINS AND BACTERIOCHLORINS CONTAINING POLYETHER
PROCEDE DE PRODUCTION DE CHLORINES ET BACTERIOCHLORINES CONTENANT UN POLYETHER

(30) Priorität: 05.07.1996 DE 19627164
(43) Veröffentlichungstag der Anmeldung: 21.04.1999
(73) Patentinhaber: Deutsches Krebsforschungszentrum Stiftung des öffentlichen Rechts, 69120 Heidelberg (DE)
(72) Erfinder: SINN, Hannsjörg, D-69168 Wiesloch (DE); SCHRENK, Hans-Hermann, D-67378 Zeiskam (DE); KAUS, Michael, D-69118 Heidelberg (DE); MAIER-BORST, Wolfgang, D-69221 Dossenheim (DE); STEHLE, Gerd, D-69123 Heidelberg (DE)
(74) Vertreter: Schüssler, Andrea, Dr.
(86) Internationale Anmeldenummer: DE9701438
(87) Internationale Veröffentlichungsnummer: WO9801156

(56) Entgegenhaltungen:
- WO-A-95/10522
- WO-A-95/29915
- DE-A- 4 017 439
- H.W. WHITLOCK ET AL.: "DIIMIDE REDUCTION OF PORPHYRINS" JOURNAL OF THE AMERICAN CHEMICAL SOCIETY, Bd. 91, Nr. 26, 17.Dezember 1969, DC US, Seiten 7485-7489, XP002053835

## Beschreibung

Die Erfindung betrifft ein Verfahren zur Herstellung Polyether-aufweisender Chlorine und Bakteriochlorine, solche und ihre Verwendung.

Es ist bekannt, Polyether-aufweisende Chlorine und Bakteriochlorine zur Behandlung von Tumoren in der photodynamischen Therapie zu verwenden. Diese werden hergestellt, indem zunächst Chlorine und Bakteriochlorine aus Porphyrinen synthetisiert werden. Nach Isolierung und Reinigung werden an die Chlorine und Bakteriochlorine Polyether gebunden. Die erhaltenen Polyether-aufweisenden Chlorine und Bakteriochlorine werden dann isoliert und gereinigt. Die mehrfachen lsolierungs- und Reinigungsschritte machen das Verfahren sehr aufwendig und teuer. Ferner können die Polyether-aufweisenden Chlorine und Bakteriochlorine nur in geringen Mengen und schlechter Ausbeute hergestellt werden.

Der vorliegenden Erfindung liegt somit die Aufgabe zugrunde, ein Verfahren bereitzustellen, mit dem Polyether-aufweisende Chlorine und Bakteriochlorine ohne vorstehende Nachteile hergestellt werden können.

Erfindungsgemäß wird dies durch die Gegenstände in den Patentansprüchen erreicht.

Gegenstand der vorliegenden Erfindung ist somit ein Verfahren zur Herstellung von Polyether-aufweisenden Chlorinen und/oder Bakteriochlorinen, das die folgenden Schritte umfaßt:
(a) Binden eines Polyethers an ein Porphyrin und
(b) Umsetzen des Polyther-aufweisenden Porphyrins mit einem Reduktionsmittel.

Der Audruck "Porphyrin" umfaßt Verbindungen jeglicher Art, die das makrocyclische Tetrapyrrolgerüst aufweisen. Vertreter dieser Verbindungen sind Porphyrine, die eine oder mehrere, vorzugsweise 4, funktionelle Gruppen, z.B. Säuregruppen, wie Carbonsäure- und/oder Sulfonsäure-Gruppen, aufweisen. Beispiele solcher sind o-, m- und/oder p-Tetracarboxyphenylporphin (TCCP) und o-, m- und/oder p-Tetrasulfophenylporphin. Günstig ist es, wenn das Porphyrin vor seiner Umsetzung mit dem Polyether aktiviert wird. Eine solche Aktivierung kann z.B. die Umwandlung vorstehender Säuregruppen in ein reaktiveres Derivat, z.B. Säurehalogenid, wie Säurechlorid oder -bromid, sein.

In Schritt (a) des erfindungsgemäßen Verfahrens wird an das Porphyrin ein Polyether gebunden, wodurch ein Polyether-aufweisendes Porphyrin erhalten wird. Die Bindung kann kovalent sein. Beispielsweise kann der Polyether an funktionelle Gruppen des Porphyrins gebunden werden. Der Polyether weist vorzugsweise ein Molekulargewicht von 100 bis 20 000 Dalton, besonders bevorzugt 2000 bis 5000 Dalton und ganz besonders bevorzugt ca. 5000 Dalton auf. Ferner kann der Polyether an der endständigen Hydroxylgruppe mit einer C₁-C₁₂-Alkylgruppe, insbesondere Methylgruppe, verethert oder verestert sein. Vertreter der Polyether sind Polyethylenglykole, wie Methoxypolyethylenglykol und Amino-Q-Methoxypolyethylenglykol (AminoPEG). Es können mehrere Polyether, vorzugsweise vier, an das Porphyrin gebunden werden. Die Polyether können gleich oder verschieden voneinander sein. Werden mehrere Polyether an das Porphyrin gebunden, beträgt die Summe der Molekulargewicht der Polyether vorzugsweise 8 000 bis 50 000 Dalton.

In Schritt (b) des erfindungsgemäßen Verfahrens erfolgt die Umsetzung des Polyether-aufweisenden Porphyrins von Schritt (a) mit einem Reduktionsmittel. Hierzu muß das Porphyrin weder isoliert noch gereinigt werden. Die Schritte (a) und (b) können daher in einer Eintopf-Reaktion erfolgen. Als Reduktionsmittel ist jedes Mittel geeignet, das eine Reduktion des Porphyrins zu einem Chlorin, d.h. Dihydroporphyrin, insbesondere 7,8-Dihydroporphyrin, und/oder Bakteriochlorin, d.h. Tetrahydroporphyrin, insbesondere 7,8,17,18-Tetrahydroporphyrin, bewirken kann. Beispiele solcher Reduktionsmittel sind Hydrazide, wie Toluolsulfonsäurehydrazid.

Die Schritte (a) und (b) des erfindungsgemäßen Verfahrens werden vorzugsweise in einem organischen, polaren, mit Wasser mischbaren Lösungsmittel, z.B. Dioxan oder ein Gemisch von Dioxan und Ethylentriamin, durchgeführt. Dies hat z.B. den Vorteil, daß Wasser oder wäßrige Lösungen zugegeben werden können und eine Ultrafiltration zur Abtrennung von unerwünschten Begleitstoffen erfolgen kann.

Nach Schritt (b) des erfindungsgemäßen Verfahrens weist der Reaktionsansatz Polyether-aufweisende Chlorine und/oder Bakteriochlorine auf. Die Zusammensetzung des Reaktionsansatzes hängt von dem Reduktionsmittel, seiner Menge und der Reaktionszeit ab. Dies kann in üblicher Weise bestimmt werden.

Vorzugsweise wird nach Schritt (b) des erfindunggemäßen Verfahrens ein Isolierungsschritt durchgeführt, der die Aufnahme des Reaktionsansatzes in Wasser, z.B. bidestilliertem Wasser, Puffer oder einem schwach reduzierenden wäßrigen Medium, wie eine Natrium-Ascorbat-Lösung, sowie nachfolgende Ultrafiltration umfassen kann. Polyether-aufweisende Chlorine sind in Wasser stabil. Dagegen werden Polyether-aufweisende Bakteriochlorine in Wasser zu den entsprechenden Polyether-aufweisenden Chlorinen autoxidiert. Auf diese Weise kann gebildetes Polyether-aufweisendes Bakteriochlorin zum entsprechenden Chlorin umgewandelt weden. In einem schwach reduzierenden, wäßrigen Medium sind sowohl Polyether-aufweisende Chlorine als auch Polyether-aufweisende Bakteriochlorine stabil. Störende Begleitstoffe, z.B. nicht umgesetzte Polyether, können durch eine Ultrafiltration abgetrennt werden. Die Ultrafiltration weist den Vorteil auf, daß sie leicht durchführbar ist. Die Trennung eines nach der Ultrafiltration vorliegenden Gemisches von Polyether-aufweisenden Chlorinen und Bakteriochlorinen kann in üblicher Weise, z.B. durch chromatographische Verfahren, erfolgen.

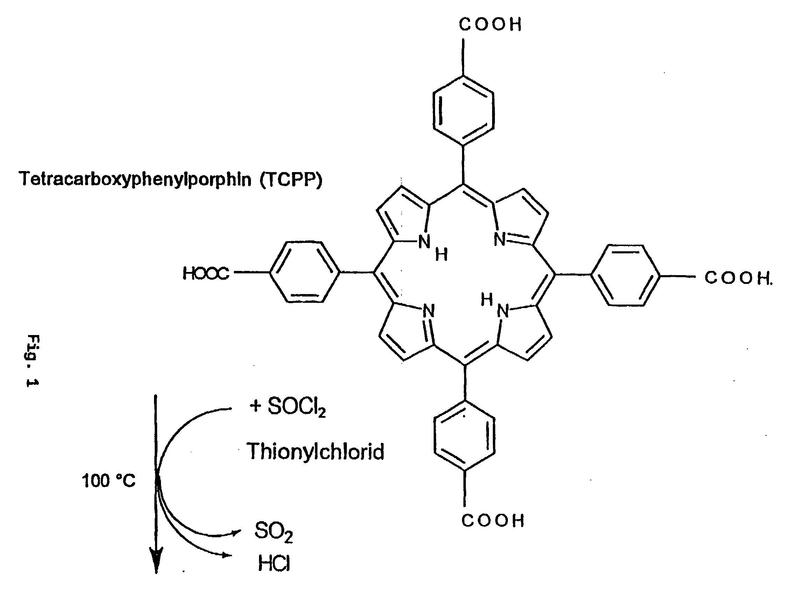

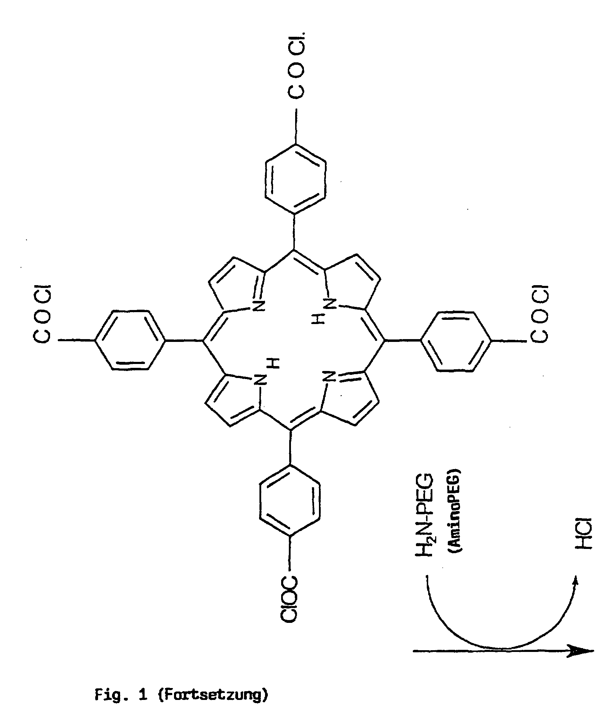

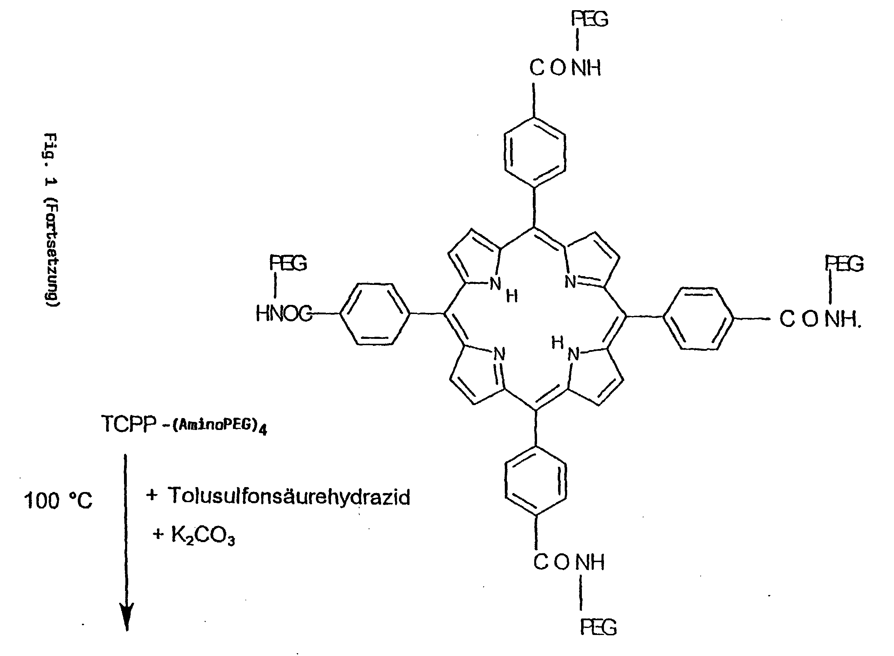

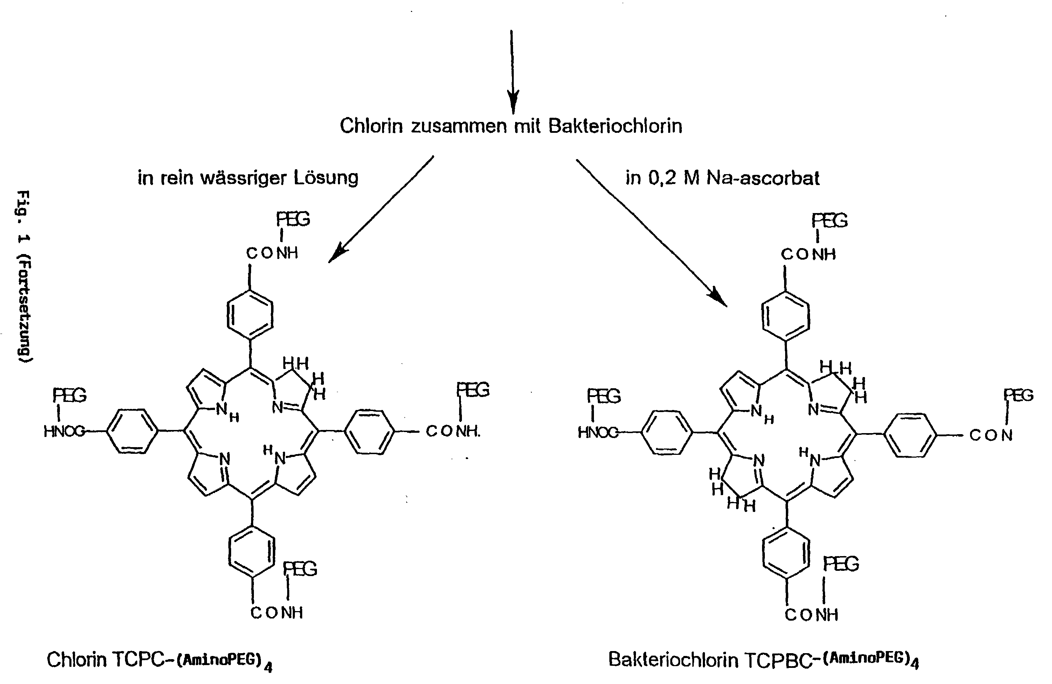

Ein bevorzugtes erfindungsgemäßes Verfahren ist in Fig. 1 dargestellt.

Das erfindungsgemäße Verfahren zeichnet sich dadurch aus, daß es einen geringen Zeit-, Material- und Arbeitsaufwand erfordert. Ferner laufen die Reaktionen praktisch quantitativ ab, so daß die Polyether-aufweisenden Chlorine und Bakteriochlorine nahezu verlustfrei, d.h. in hohen Ausbeuten, aus den Porphyrinen hergestellt werden können. Des weiteren liegen die Polyether-aufweisenden Chlorine und Bakteriochlorine bereits in einer tumorgängigen Form vor und können in kurzer Zeit applikationsfertig bereitgestellt werden. Somit eignet sich das erfindungsgemäße Verfahren bestens zu Herstellung von Polyether-aufweisenden Chlorinen und Bakteriochlorinen, die für die photodynamische Therapie von Tumoren verwendet werden können.

Erfindungsgemäß hergestellte Polyether-aufweisende Chlorine und Bakteriochlorine sind ebenfalls ein Gegenstand der vorliegenden Erfindung.

### Kurze Beschreibung der Zeichnung:

- Fig. 1: zeigt die erfindungsgemäße Herstellung von Polyether-aufweisenden Chlorinen und Bakteriochlorinen. Hierzu wird TCPP mit Thionylchlorid umgesetzt, um die Carbonsäure-Gruppendes TCPP in die Säurechlorid-Form zu überführen. Anschließend erfolgt die Bindung von 4 AminoPEG und der Reduktion mit Toluolsulfonsäurehydrazid, wodurch ein Polyether-aufweisendes Chlorin und Bakteriochlorin erhalten wird.

Die Erfindung wird durch das Beispiel erläutert:

### Beispiel: Herstellung von Polyether-aufweisenden Chlorin und Bakteriochlorin.

Die Herstellung ist in Fig. 1 gezeigt.

### 1. Herstellung des Säurechlorids von TCPP:

40 mg p-Tetracarboxyphenylporphin wurden in einem 50 ml Rundkolben mit NS 29 vorgelegt und mit 5 ml Thionylchlorid (SOCl₂) versetzt. Nach dem Aufsetzen eines Rückflußkühlers wurde die Mischung in einem Siliconölbad auf etwa 100°C erhitzt, wobei schon nach kurzer Zeit eine intensiv grüne Lösung entstand. Zur Vervollständigung der Reaktion wurde die Mischung etwa 30 Minuten unter Rückfluß erwärmt. Anschließend wurde das überschüssige SOCl₂ am Rotationsverdampfer entfernt.

### 2. Umsetzung des Säurechlorids von TCPP mit Amino-Ω-Methoxypolyethylenglykol (AminoPEG)

Zu dem trockenen Rückstand des vorstehenden Säurechlorids wurden 1 g AminoPEG, gelöst unter leichter Erwärmung in 10 ml 1,4-Dioxan, und etwa 1 ml Ethylentriamin zugegeben und während 30 Minuten auf 130°C erwärmt, wobei eine intensiv rote Lösung entstand. Es wurde TCPP-(AminoPEG)₄ erhalten.

### 3. Umsetzung des TCPP-(AminoPEG)₄ zum entsprechenden Chlorin und Bakteriochlorin

Nach dem Abkühlen der TCPP-(AminoPEG)₄-Lösung wurde diese in einen 150 ml Dreihalskolben (Sulfierkolben mit 2 NS 14,5 und einem NS 29 Schliffen) überführt und etwa 0,5 g K₂CO₃ sowie etwa 100 mg Toluolsulfonsäurehydrazid (TSH) hinzugegeben. Das Reaktionsgefäß wurde mit einem Rückflußkühler und einer Gaseinleitungskapillare versehen. Bevor das Reaktionsgemisch erwärmt wurde, wurde zur Verdrängung der Luft etwa 20 Minuten mit Stickstoff gespült. Anschließend wurde die Reaktionslösung auf eine Temperatur von etwa 100°C erwärmt. Nach etwa 1,5 Stunden wurde über den dritten Seitenhals erneut 100 mg TSH zugegeben. Diese Zugabe wurde nach weiteren 1,5 Stunden wiederholt. Nach einer Gesamtreaktionszeit von etwa 6 Stunden war die Umsetzung zum Chlorin (TCPC) beendet, wobei ein Teil des TCPP-(AminoPEG)₄ in die Bakteriochlorinform (TCPBC) überführt worden war. Die Bildung des TCPC und TCPBC kann durch gelegentliche Probennahmen und deren spektroskopischen Untersuchungen verfolgt werden.

### 4. Reinigung bzw. Überführung in reines Chlorin

Nach Abkühlung der Reaktionslösung wurde diese zur Bereitstellung von Chlorin in 1 l bidest. Wasser eingebracht und die störenden Begleitstoffe durch eine Ultrafiltration (Amicon YM 10) abgetrennt. Zum Erhalt des Bakteriochlorins wurde in schwach reduzierendem Medium (0,1 M Na-Ascorbat) gearbeitet.

## Patentansprüche

1. Verfahren zur Herstellung von Polyether-aufweisenden Chlorinen und/oder Bakteriochiorinen, umfassend die folgenden Schritte:
(a) Binden eines Polyethers an ein Porphyrin und
(b) Umsetzen des Polyether-aufweisenden Porphyrins mit einem Reduktionsmittel.

2. Verfahren nach Anspruch 1, **dadurch gekennzeichnet, daß** das Porphyrin mindestens eine Säuregruppe aufweist.

3. Verfahren nach Anspruch 2, **dadurch gekennzeichnet, daß** das Porphyrin o-, m- und/oder p-Tetracarboxyphenylporphin bzw. o-, m- und/oder p-Tetrasulfophenylporphin ist.

4. Verfahren nach einem der Ansprüche 1-3, **dadurch gekennzeichnet, daß** der Polyether ein Molekulargewicht von 100 bis 20 000 Dalton aufweist.

5. Verfahren nach einem der Ansprüche 1-4, **dadurch gekennzeichnet, daß** der Polyether ein Polyethylenglykol ist.

6. Verfahren nach einem der Ansprüche 1-5, **dadurch gekennzeichnet, daß** mehrere Polyether an das Porphyrin gebunden werden.

7. Verfahren nach einem der Ansprüche 1-6, **dadurch gekennzeichnet, daß** die Reduktion mit einem Hydrazid erfolgt.

8. Verfahren nach einem der Ansprüche 1-7, **dadurch gekennzeichnet, daß** die Schritte (a) und-(b) in einem organischen, polaren Lösungsmittel durchgeführt werden.

9. Verfahren nach einem der Ansprüche 1-8, **dadurch gekennzeichnet, daß** nach Schritt (b) ein Isolierungssschritt erfolgt.

10. Polyether-aufweisendes Chlorin bzw. Bakteriochlorin mit den Strukturformeln 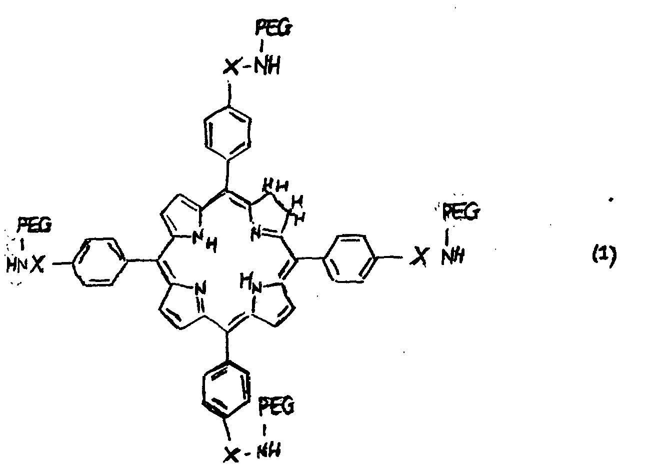 oder 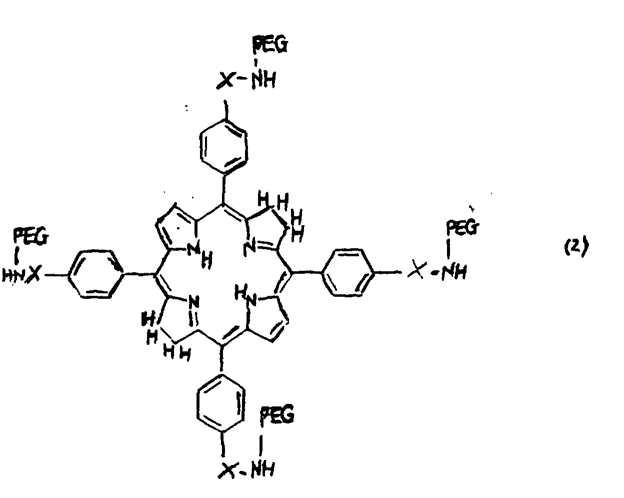 wobei X CO oder SO₂ bedeutet, und PEG Polyether, insbesondere Polyethylenglykol oder Derivate davon bedeutet.

11. Verwendung eines Polyether-aufweisenden Chlorins oder Bakteriochlorins nach Anspruch 10 zur Herstellung einen Pharmazeutische Zusammensetzung die in den photodynamischen Therapie verwendet werden kann.

## Claims

1. A method of producing polyether-containing chlorins and/or bacteriochlorins, comprising the steps of:
(a) binding a polyether to a porphyrin and
(b) reacting the polyether-containing porphyrin with a reducing agent.

2. The method according to claim 1, **characterized in that** the porphyrin has at least one acid group.

3. The method according to claim 2, **characterized in that** the porphyrin is o-, m- and/or p-tetracarboxyphenylporphin or o-, m- and/or p-tetrasulfophenylporphin.

4. The method according to any of claims 1 to 3, **characterized in that** the polyether has a molecular weight of 100 to 20,000 dalton.

5. The method according to any of claims 1 to 4, **characterized in that** the polyether is a polyethylene glycol.

6. The method according to any of claims 1 to 5, **characterized in that** several polyethers are bound to the porphyrin.

7. The method according to any of claims 1 to 6, **characterized in that** the reduction is made using a hydrazide.

8. The method according to any of claims 1 to 7, **characterized in that** steps (a) and (b) are carried out in an organic polar solvent.

9. The method according to any of claims 1 to 8, **characterized in that** step (b) is followed by an isolation step.

10. The polyether-containing chlorin or bacteriochlorin comprising the structural formulae 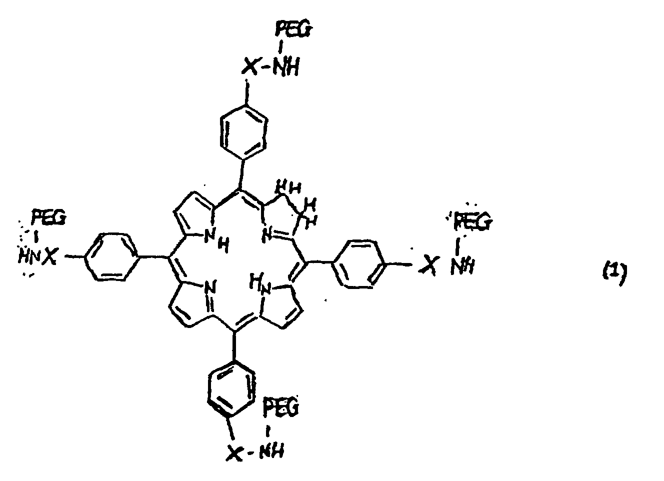 or 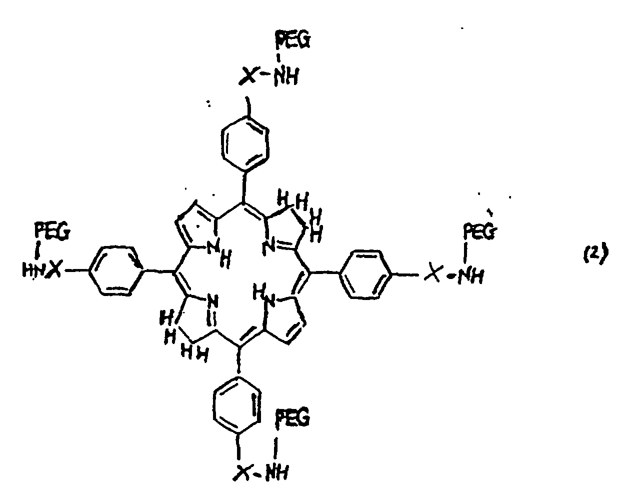 wherein X is CO or SO₂, and
PEG is polyether, in particular polyethylene glycol or derivatives thereof.

11. Use of a polyether-containing chlorin or baeteriochlorin according to claim 10 for the production of a pharmaceutical composition which can be used for the photodynamic therapy.

## Revendications

1. Procédé de production de chlorines et/ou de bactériochlorines contenant un polyéther, qui comprend les étapes suivantes :
(a) liaison d'un polyéther à une porphyrine et
(b) réaction de la porphyrine contenant un polyéther avec un agent réducteur.

2. Procédé suivant la revendication 1, **caractérisé en ce que** la porphyrine porte au moins un groupe acide.

3. Procédé suivant la revendication 2, **caractérisé en ce que** la porphyrine consiste en o-, m- et/ou p-tétracarboxyphénylporphine ou en o-, m- et/ou p-tétrasulfonylphénylporphine.

4. Procédé suivant l'une des revendications 1 à 3, **caractérisé en ce que** le polyéther présente un poids moléculaire de 100 à 20 000 daltons.

5. Procédé suivant l'une des revendications 1 à 4, **caractérisé en ce que** le polyéther est un polyéthylène-glycol.

6. Procédé suivant l'une des revendications 1 à 5, **caractérisé en ce que** plusieurs polyéthers sont liés à la porphyrine.

7. Procédé suivant l'une des revendications 1 à 6, **caractérisé en ce que** la réduction est conduite avec un hydrazide.

8. Procédé suivant l'une des revendications 1 à 7, **caractérisé en ce que** les étapes (a) et (b) sont conduites dans un solvant organique polaire.

9. Procédé suivant l'une des revendications 1 à 8, **caractérisé en ce qu'**une étape d'isolement est conduite après l'étape (b).

10. Chlorine ou bactériochlorine contenant un polyéther, répondant aux formules structurales 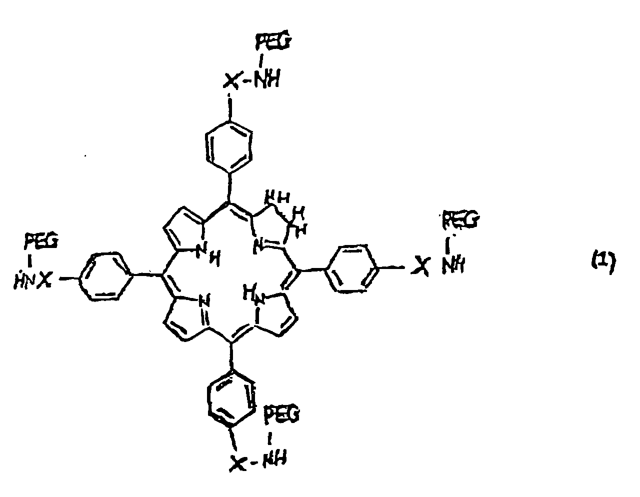 ou 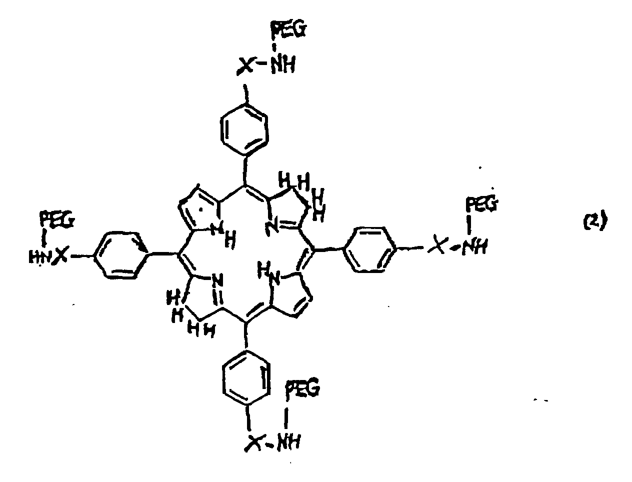 où X représente CO ou SO₂, et
PEG désigne un polyéther, en particulier un polyéthylène-glycol ou ses dérivés.

11. Utilisation d'une chlorine ou d'une bactériochlorine contenant un polyéther suivant la revendication 10 pour la préparation d'une composition pharmaceutique qui peut être utilisée en thérapie photodynamique.
